# EUROPÄISCHE PATENTANMELDUNG

(11) **EP 1 304 160 A1**
(43) Veröffentlichungstag der Anmeldung: **23.04.2003**
(21) Anmeldenummer: 02090359.7
(22) Anmeldetag: 21.10.2002
(51) Int. Cl.: B01J 13/02

(54) **Stabilisierte Mischungen von Liposomen und Emulsionen**

(30) Priorität: 19.10.2001 DE 10152145
(71) Anmelder: Novosom AG, 06120 Halle (DE)
(72) Erfinder: Panzner Steffen, 06108 Halle (DE); Endert Gerold, 06114 Halle (DE); Lutz Silke, 06114 Halle (DE)
(74) Vertreter: Ziebig, Marlene, Dr. Dipl.-Chem.

(57) **Zusammenfassung**

Liposomen oder Tröpfchen einer o/w-Emulsion können durch Aufbringen einer Hüllschicht so stabilisiert werden, dass sie mit anderen Liposomen oder Emulsionen stabile Mischungen bilden, wobei die Hüllschichten aus wasserlöslichen Polymeren, die über eine Vielzahl funktioneller Gruppen verfügen, bestehen.

## Beschreibung

Die vorliegende Erfindung betrifft Mischungen kolloidal gelöster Partikelsorten, die durch Hüllschichten aus Polymeren stabilisiert sind.

Eine solche Stabilisierung ist immer dann notwendig, wenn die einzelnen Komponenten miteinander nicht stabil mischbar sind, etwa wenn es zur Flockung, zur Auflösung oder zur Verschmelzung von Teilchen kommt und diese Prozesse nicht erwünscht sind.

Kolloidal vorliegende Teilchen, insbesondere Liposomen oder o/w-Emulsionen können durch elektrostatische Abstossung oder durch sterische Hinderung stabilisiert werden. Beide Möglichkeiten sind Grundlagen der Kolloidchemie und als solche bekannt. Neuere Methoden und Komponenten zur sterischen Stabilisierung von Emulsionen betreffen nichtionische Emulgatoren, wie etwa Polyoxyethylensorbitanether (Tween®). Diese Stoffe sind auch zur Stabilisierung von festen Nanopartikeln in Körperflüssigkeiten geeignet (WO 98/56361 oder US 5,601,835). Liposomen können wirksam durch kovalente Anbindung von Polyethylenglykol (PEG) stabilisiert werden.

Die bekannten Verfahren haben jedoch mehrere Nachteile. Eine Stabilisierung aufgrund elektrostatischer Abstossung ist nur möglich, wenn beide Klassen von Kolloidteilchen die gleiche Ladung tragen. Ein unterschiedliches Vorzeichen der Ladung führt zur Flockung. Das ist etwa der Fall, wenn kationische geladene Liposomen, wie sie für die Transfektion von Zellen benutzt werden, mit anderen Kolloiden, etwa denen des Serums, in Kontakt kommen. Im System Liposom/Emulsion tritt gewöhnlich eine Leckage der Liposomen und damit verbunden ein Austritt von eingeschlossenen Wirkstoffen auf. Die Verwendung PEG-modifizierter Lipide umgeht dieses Problem, allerdings sind diese Substanzen sehr teuer. Ihr Einsatz beschränkt sich daher auf pharmazeutische Anwendungen.

Aufgabe der Erfindung ist es daher, eine kostengünstige Methode zur Stabilisierung von Mischungen aus verschiedenen Kolloiden, insbesondere von Liposomen, o/w-Emulsionen in Mischung mit anderen Liposomen, o/w-Emulsionen und/oder w/o-Emulsionen bereitzustellen.

Die vorliegende Erfindung löst dieses technische Problem durch Bereitstellung von Mischungen, umfassend mindestens zwei kollodial gelöste Partikelsorten, wobei mindestens eine der Partikelsorten eine Hüllschicht aus Polymeren aufweist.

Die DE 198 52 928 und die WO 00/28972 sowie die DE 100 10 264 und die WO 01/64330 beschreiben verschiedene Verfahren, die allesamt zu einer Umhüllung von kolloidalen Teilchen mit in sich stabilen Schichten aus Polymeren führen. Weitere Methoden zur Herstellung solcher Schichten auf verschiedenen kolloidalen Templaten sind in der US5512268 oder der US5916596 sowie in der WO00/03797 gegeben. Die benannten Druckschriften sind in den Offenbarungsgehalt der vorliegenden Erfindung mit aufgenommen.

Überraschenderweise wurde gefunden, dass solche Hüllen zur Stabilisierung von Mischungen verschiedener Kolloide geeignet sind. So lassen sich verschiedenartig geladene Liposomen nach Ausbildung der Hüllschichten miteinander mischen, ohne dass es zu einer Fusion und damit zum Austausch oder zur Freisetzung der Inhaltsstoffe kommt. Die Herstellung solcher Mischungen kann beispielsweise notwendig sein, weil sich einzelne Inhaltsstoffe in definiert geladenen Liposomen mit hoher Effizienz verpacken lassen. Vorteilhaft werden Nukleinsäuren besonders gut in kationische Liposomen eingeschlossen. Die erfinderische Lehre läßt sich beispielsweise zur Herstellung von Cremes oder Lotionen einsetzen, in denen zusätzlich Liposomen als Träger von Wirkstoffen verwendet werden.

Eine bevorzugte Ausführung der Erfindung besteht in der Stabilisierung von Liposomen in Gegenwart von Emulsionen. Hier wird oft eine Permeabilisierung der Liposomen und der Austritt von Wirkstoffmolekülen aus deren Innern oder auch die Verschmelzung beider Kolloide beobachtet. In beiden Fällen erfüllen die Liposomen ihre eigentliche Funktion - den Schutz des eingeschlossenen Wirkstoffs - nicht mehr. In sich stabile Hüllschichten wie die oben benannten verhindern eine Verschmelzung der Liposomen mit dem anderen Kolloid und wirken daher stabilisierend. Dabei ist es vorteilhafterweise unerheblich, ob die Liposomen oder das andere Kolloid mit der Hüllschicht umgeben werden. Eine besonders vorteilhafte Ausführung ist gegeben, wenn die aufgebrachten Hüllschichten sich auf den Tröpfchen einer o/w-Emulsion befinden und die sonst benutzten Emulgatoren ersetzen oder deren eingesetzte Menge verringern. Bevorzugt ist, dass die Polymerschichten mindestens zwei wasserlösliche Polymere umfassen. Die Polymere weisen bevorzugt eine Vielzahl funktioneller Gruppen auf.

In einer weiteren Ausführungsform der Erfindung können Liposomen in eine w/o-Emulsion eingebracht werden, ohne ihre Integrität zu verlieren. In einer bevorzugten Variante werden die Liposomen mit den oben benannten Hüllschichten umgeben. Sie lassen sich dann insbesondere als Zusatz zu Salben oder Suppositorien einsetzen, in denen Liposomen als Träger von Wirkstoffen verwendet werden. Bevorzugt ist weiterhin, dass eine der Partikelsorten Liposomen sind, die mit Polymerschichten stabilisiert werden. In einer weiteren bevorzugten Ausführungsform ist mindestens eine der Partikelsorten eine o/w-Emulsion, die mit Polymerschichten stabilisiert wird.

Liposomen wie auch Emulsionen werden zur Formulierung von Wirkstoffen verwendet oder stellen selbst einen Wirkstoff einer Zubereitung dar. Wirkstoffe und Anwendungsmöglichkeiten sind dem Fachmann bekannt.

Die folgenden Beispiele sollen die erfinderische Lehre illustrieren, ohne sie auf die genannten Ausführungsformen zu beschränken.

### Abkürzungen

- DSPG: Distearoyl-Phosphatidylglycerol
- DSPC: Distearoyl-Phosphatidylcholin
- DC-Chol: N,N-Dimethyldiaminoethyl-N'carbamoylcholesterol
- Chol: Cholesterol
- PAA: Polyallylamin
- PAS: Polyacrylsäure

### Beispiel 1

### Verhinderung der Aggregation von Liposomen

### Herstellung der Liposomen

60mol-% DSPG und 40mol-% Chol (Liposom A) bzw. 60mol-% DSPC und 40mol-% DC-Chol (Liposom B) werden in einem Gemisch aus Isopropanol und Chloroform (2/1) gelöst und unter Vakuum bis zur Trockene eingedampft. Die beiden Lipidfilme werden anschließend in soviel Puffer (10mM Na-Acetat, 100mM NaCl, pH 4) rehydratisiert, dass eine Lipidkonzentration von 15mg/ml erhalten wird. Die Suspensionen werden mehrmals durch isopore Polykarbonatmembranen mit einer Porengröße von 200nm extrudiert.

### Beschichtung der Liposomen mit Chitosan und Alginat

Chitosan und Alginat werden in einer Konzentration von 1mg/ml in Puffer (10mM Na-Acetat, pH 4) gelöst. Die Liposomen A werden in dem selben Puffer auf eine Lipidkonzentration von 0,15mg/ml verdünnt. Die unten angegebenen Mengen Polymer werden sukzessive zu den Liposomen A gegeben:

| Zyklus | Polymer | µg Polymer/mg Lipid |
|---|---|---|
| 1 | Chitosan | 87 |
| 2 | Alginat | 36 |
| 3 | Chitosan | 48 |
| 4 | Alginat | 81 |
| 5 | Chitosan | 83 |

Sie binden hinreichend vollständig an die Liposomen, so dass keine weiteren Wasch- oder Trennschritte erforderlich sind.

### Aggregationsverhalten der Strukturen

Liposomen A, B oder die Nanokapseln werden mittels dynamischer Lichtstreuung vermessen und ergeben Partikeldurchmesser von 165nm (Liposom A), 172nm (Liposom B) und 315nm (Nanokapseln). Die Mischung Liposom A und B führt zur sofortigen Bildung von grossen Aggregaten. Diese Aggregation wird bei der Mischung aus A hergestellten Nanokapseln mit den Liposomen B nicht mehr beobachtet, die Partikeldurchmesser bleiben konstant.

### Beispiel 2

### Stabilisierung von Liposomen in o/w-Emulsionen

### Herstellung der Liposomen

Liposomen A werden wie in Beispiel 1 hergestellt. Die Hydratisierung erfolgt in einem Puffer enthaltend 10mM HEPES, 50mM NaCl, 100mM Carboxyfluorescein, pH 7,5), so dass eine Lipidkonzentration von 15mg/ml erhalten wird. Die Suspension wird wie oben extrudiert.
Nicht eingeschlossenes Carboxyfluorescein wird durch Gelfiltration abgetrennt.

### Beschichtung der Liposomen mit PAA und PAS (bei pH 7,5)

PAA und PAS werden in einer Konzentration von 1mg/ml in Puffer (10mM Hepes, 100mM NaCl, pH 7,5) gelöst. Die Liposomen werden in dem selben Puffer auf eine Lipidkonzentration von 0,15mg/ml verdünnt. Die Beschichtung der Liposomen erfolgt wie in Beispiel 1 mit folgenden Polymermengen:

| Schicht | Polymer | µg Polymer/mg Lipid |
|---|---|---|
| 1 | PAA | 60 |
| 2 | PAS | 53 |
| 3 | PAA | 74 |
| 4 | PAS | 107 |

Die Liposomen werden durch Tangentialdialyse bis auf 20mg/ml aufkonzentriert.

### Analysen zur Stabilität der Strukturen

Die Liposomen A und die daraus hergestellten Nanokapseln besitzen einen Partikeldurchmesser von 194nm (Liposomen A) und 237nm (Nanokapseln).
1ml der Liposomen A bzw. 1ml der daraus hergestellten Nanokapseln werden mit je 5ml einer o/w-Emulsion (Lipovenös, ) gemischt und für 24h inkubiert.
Die Mischung von Liposomen A und der Emulsion fluoresciert danach mit mehr als 30% der maximalen Intensität. Die Mischung der Nanokapseln und der emulsion fluoresciert mit weniger als 15% der maximalen Intensität.

### Beispiel 3

### Stabilisierung von Liposomen in o/w-Emulsionen

Liposomen werden wie in Beispiel 2 hergestellt und mit einer Konzentration von 0,15mg/ml in Puffer (10mM Na-Acetat, 50mM Glutaraldehyd, pH4,0) gelöst. Die Beschichtung der Liposomen erfolgt wie in Beispiel 1 mit Chitosan und Natriumalginat, wobei eine zusätzliche Schicht Alginat (150µg Alginat/mg Lipid) aufgebracht wird. Nach Abschluß der Polymerzugabe werden die Liposomen für 3h bei 40°C inkubiert, danach wird Trishydroxyethyl-aminomethan mit einer Endkonzentration von 500mM und einem pH-Wert von 8 zugegeben. Die Nanokapseln werden wie in Beispiel 2 aufkonzentriert.

### Analysen zur Stabilität der Strukturen

Die Liposomen A und die Nanokapseln werden mittels dynamischer Lichtstreuung vermessen und ergeben einen Partikeldurchmesser von 194nm (Liposomen A) und 296nm (Nanokapseln).
1ml der Liposomen A bzw. 1ml der daraus hergestellten Nanokapseln werden wie in Beispiel 2 mit je 5ml einer o/w-Emulsion (Lipovenös, ) gemischt, für 24h inkubiert und anschließend im Fluorimeter vermessen.
Die Mischung der Liposomen A mt der Emulsion fluoresciert mit mehr als 30% der maximalen Intensität, die der Nanokapseln mit weniger als 20% der maximalen Intensität der Probe.

### Beispiel 4

### Stabilisierung von Liposomen in w/o-Emulsionen

Liposomen A werden wie Beispiel 2 hergestellt und mit Polymerschichten umhüllt. Eine weitere Polymerschicht enthält 105µg PAA/mg Lipid. Anschließend werden die Partikel durch Tangentialdialyse aufkonzentriert.
0,5ml der konzentrierten Nanokapseln werden mit einem Ultraturrax in 5g Sojaöl dispergiert. 200mg Sojalecithin und 50mg DPPG werden in 200µl Ethanol gelöst und langsam unter weiterem Rühren zur Dispersion gegeben.

Die erhaltene Suspension ist lagerstabil, eine Freisetzung des Markers in die Ölphase ist nicht zu beobachten.

### Beispiel 5

### Stabilisierung einer Emulsion in Gegenwart von Liposomen

Liposomen A werden wie in Beispiel 2 hergestellt und von nicht eingeschlossenem Carbxyfluorescein getrennt, aber nicht weiter stabilisiert.

### Beschichtung einer Emulsion mit PLL und PAS (bei pH 7,5)

PLL und PAS werden in einer Konzentration von 1mg/ml in Puffer (10mM Hepes, pH 7,5) gelöst. Eine o/w- Emulsion (Lipovenös, ) wird in dem selben Puffer auf 0,1mg/ml verdünnt. Folgende Mengen Polymer werden zur Emulsion sukzessive zugegeben:

| Zyklus | Polymer | µg Polymer/mg Lipid |
|---|---|---|
| 1 | PLL | 85 |
| 2 | PAS | 30 |
| 3 | PLL | 120 |
| 4 | PAS | 40 |
| 5 | PLL | 100 |
| 6 | PAS | 30 |

Die jeweils eingesetzte Menge Polymer bindet hinreichend vollständig an die Partikeloberfläche, so dass keine Trennungen erforderlich sind.
Die Emulsion kann durch Tangentialdialyse oder Vakuumtrocknung wieder aufkonzentriert werden.

### Analyse zur Stabilität der Strukturen

2ml Puffer, Ausgangsemulsion oder stabilisierte Emulsion (12mg/ml) werden mit 2ml der Liposomen gemischt und für 24h inkubiert.
2µl des jeweiligen Ansatzes werden mit 5ml Puffer (10mM HEPES pH7,5) verdünnt, anschließend wird die Fluoreszensintensität der Probe bestimmt.
Die Mischung der unbehandelten Emulsion mit den Liposomen fluoresciert danach mit mehr als 25% der maximalen Intensität. Die Mischung der polymerstabilisierten Emulsion und der Liposomen fluoresciert mit weniger als 15% der maximalen Intensität.

## Patentansprüche

1. Mischung, umfassend mindestens zwei kolloidal gelöste Partikelsorten, **dadurch gekennzeichnet, dass** mindestens eine der Partikelsorten eine Hüllschicht aus Polymeren aufweist.

2. Mischung nach Anspruch 1, **dadurch gekennzeichnet, dass** die Polymerschicht mindestens zwei wasserlösliche Polymere umfasst.

3. Mischung nach einem der Ansprüche 1 oder 2 , **dadurch gekennzeichnet, dass** mindestens eine der Partikelsorten Liposomen mit Polymerschichten sind.

4. Mischung nach einem der Ansprüche 1 bis 3, **dadurch gekennzeichnet, dass** mindestens eine der Partikelsorten eine o/w-Emulsion mit Polymerschichten ist.
